# EUROPÄISCHE PATENTSCHRIFT

(11) **EP 0 464 573 B1**
(45) Veröffentlichungstag und Bekanntmachung des Hinweises auf die Patenterteilung: **08.04.1998**
(21) Anmeldenummer: 91110409.9
(22) Anmeldetag: 24.06.1991
(51) Int. Cl.: A61L 15/16

(54) **Pflaster mit hohem Gehalt an weichmachenden Inhaltsstoffen**
Plaster high in softening substances
Pansement à haute teneur en substances adoucissantes

(30) Priorität: 25.06.1990 DE 4020144
(43) Veröffentlichungstag der Anmeldung: 08.01.1992
(73) Patentinhaber: LTS LOHMANN THERAPIE-SYSTEME GmbH & CO.KG, 56513 Neuwied (DE)
(72) Erfinder: Müller, Walter, Dr. Dipl.-Chem., W-5450 Neuwied 1 (DE); Minderop geb. Heidemann, Raphaela, Dr., W-8000 München 2 (DE); Teubner, Andreas, Dr., W-8068 Pfaffenhofen (DE)
(74) Vertreter: Flaccus, Rolf-Dieter, Dr.

(56) Entgegenhaltungen:
- EP-A- 0 305 758
- EP-A- 0 379 933
- DE-A- 3 843 238
- DE-A- 3 843 239

## Beschreibung

Die Erfindung betrifft eine Vorrichtung zur topischen und systemischen Verabreichung von Wirkstoffen an bzw. durch die Haut, entsprechend den gattüngsbildenden Merkmalen von Ansprüch 1.

Dermale bzw. transdermale therapeutische Systeme haben sich mittlerweile ihren festen Platz unter den Arzneiformen gesichert. Sie bestehen im einfachsten Fall aus einer Rückschicht, einer selbstklebenden wirkstoffhaltigen Matrixschicht und einer wieder entfernbaren Schutzschicht. Bei diesen sogenannten Matrixsystemen besteht die Matrixschicht neben dem bzw. den Wirkstoffen und Hilfsstoffen bevorzugt aus einem selbstklebenden Polymer bzw. einer selbstklebenden Polymermischung. Ein solches System ist z.B. in der US-PS 3,734,097 beschrieben.

Besonderes Gewicht bei diesen Systemen hat die Klebrigkeit der Matrix, da nur bei einem sicheren und reproduzierbaren Kontakt des Systems mit der Haut der Wirkstoff in der gewünschten Weise über Diffusionsprozesse auf bzw. in die Haut übertreten, diese gegebenenfalls passieren und dann systemisch wirksam werden kann.

Wird als klebende Matrixkomponente ein Polyacrylat- oder Polysiloxanharz verwendet, wird die Klebrigkeit unter anderem durch das mittlere Molekulargewicht und die Molekulargewichtsverteilung des Harzes bestimmt. Bei Klebern, die als Grundpolymer ein nichtklebendes, gummiartiges Polyisobutylen oder Styrol-Butadien-Blockpolymer enthalten, müssen zusätzliche klebrigmachende Harze zugemischt werden. Dies sind oftmals chemisch modifizierte Naturharze.

In einer ganzen Reihe von Fällen müssen zur Erzielung von genügend hohen Wirkstoffflüssen aus dem System an und durch die Haut sehr hohe Konzentrationen an Wirkstoff eingesetzt werden. Insbesondere wenn diese Wirkstoffe bei Raumtemperatur flüssig sind, haben sie einen negativen Einfluß auf die Eigenschaften der Matrix. Die Kohäsion läßt nach, und die Konsistenz der Matrix erinnert an Honig oder "Fliegenleim".

Ähnliche Probleme ergeben sich mit Wirkstoffen, die entweder aufgrund ihrer chemisch-physikalischen Eigenschaften nur sehr schwer in genügender Menge und Geschwindigkeit die Barriere der menschlichen Haut durchdringen können oder deren therapeutische Dosis relativ hoch ist.

In diesen Fällen ist man auf die Verwendung von sogenannten Penetrationsbeschleunigern angewiesen. Diese Substanzen reduzieren die Barrierefunktion der Haut und erhöhen so den maximal erreichbaren Wirkstofffluß.
Die meisten dieser Penetrationsbeschleuniger sind bei Raumtemperatur Flüssigkeiten und müssen oft in relativ hohen Mengen an die Haut abgegeben werden. Die Problematik, die sich dadurch zwangsläufig ergibt, ist die gleiche wie bei den Wirkstoffen. In hohen Konzentrationen haben sie einen nicht mehr tolerierbaren negativen Einfluß auf die Kohäsion der Matrix.

Gehört der Wirkstoff beispielsweise zu der Gruppe der Antirheumatika, müssen die Pflaster aufgrund der relativ hohen benötigten Wirkstoffdosis recht groß ausfallen. Für die meisten Wirkstoffe kann dabei eine Pflastergröße von 100 cm² nicht unterschritten werden. Bei diesen großen Pflastern kann wegen des Tragekomforts keine unelastische Folie als Rückschicht verwendet werden. Hier empfehlen sich textile Flächengebilde. Da solche Pflaster bevorzugt mehrtägig getragen werden, ist es vorteilhaft, wenn sie wasserabweisend aber wasserdampfdurchlässig ausgerüstet sind. Durch die wasserabweisende Wirkung wird verhindert, daß sie sich beim Duschen mit Wasser vollsaugen, und durch die Wasserdampfdurchlässigkeit wird der Okklusionseffekt etwas abgemildert. Bei dieser Art von Pflastern ist es auch von Vorteil, wenn eine lokal durchblutungsfördernde bzw. eine ein Wärmegefühl hervorrufende Komponente zusätzlich enthalten ist. Eine ganze Reihe dieser Substanzen z.B. Nicotinsäureester und Salicylsäuremethylester sind bei Raumtemperatur Flüssigkeiten und verstärken dadurch das Konsistenzproblem. Besonders gravierend sind die Konsistenzprobleme bei sogenannten Beutelsystemen, die den Wirkstoff in Form einer flüssigen oder halbflüssigen Zubereitung enthalten. Diese Systeme haben, wie der Name schon sagt, die Form eines Beutels, der das Wirkstoffreservoir darstellt und aus einer undurchlässigen Rückschicht und einer Membran gebildet wird. In den meisten Fällen steuert die Membran die Abgabe von Wirk- und/oder Hilfsstoffen aus den System. Zur Verankerung auf der Haut ist die Membran dieser Systeme mit einer dünnen Kleberschicht versehen. Diese Kleberschicht sättigt sich bis zur Einstellung eines Gleichgewichts mit allen zur Diffusion befähigten Inhaltsstoffen und damit auch den flüssigen Bestandteilen des Wirkstoffreservoirs mit entsprechenden Folgen für die Konsistenz des Klebers.

Da die meisten Penetrationsbeschleuniger im Vergleich zu den Wirkstoffen aus relativ kleinen Molekülen bestehen und deshalb eine im Vergleich zu den Wirkstoffen hohe Diffusionsgeschwindigkeit in der Matrix haben, wäre es bei Matrixsystemen eine Möglichkeit, die Matrix dicker zu machen, um ihre Aufnahmekapazität für Penetrationsbeschleuniger bei gleicher Konzentration zu steigern. Diese Lösung hat jedoch den Nachteil, daß dann auch größere Mengen an oftmals sehr teuerem Wirkstoff benötigt werden, da man mit der Konzentration des Wirkstoffs selbst nicht heruntergehen kann, ohne den Wirkstoffflux zu erniedrigen.

Eine andere Möglichkeit ist in der US-PS 4,746,515 der Firma Alza beschrieben. Hier besteht die Matrix aus zwei Schichten, wobei die der Haut entferntere Schicht nur Penetrationsbeschleuniger enthält. Eine solche Lösung ist allerdings nur dann möglich, wenn der Wirkstoff in der der Haut näheren Schicht oberhalb der Sättigungslöslichkeit und damit teilweise kristallin vorliegt, da ansonsten ein Konzentrationsausgleich zwischen den beiden Matrixschichten stattfinden würde.

Ist die weichmachende Komponente der Wirkstoff, so verbietet sich diese Lösung von selbst, und man muß versuchen, durch eine geeignete Formulierung eine gute Konsistenz der Matrix zu errreichen. Bei Klebern auf der Basis von z.B. Polyisobutylen oder Styrol-Butadien-Blockpolymeren kann man den weichmachenden Wirkungen durch eine andere Abmischung des Grundpolymers mit den klebrigmachenden Zusätzen etwas gegensteuern.

Bei den Polyacrylaten bzw. Polysiloxanen kann die Konsistenz der Matrix prinzipiell durch eine andere Molekulargewichtsverteilung im Kleber verbessert werden. Dies bedeutet jedoch eine Neusynthese eines Klebers und, daß nicht auf bewährte, sich auf dein Markt befindliche, toxikologisch gut untersuchte Kleber-formulierungen zurückgegriffen werden kann. Eine andere Möglichkeit besteht darin, Kleber nachträglich chemisch zu modifizieren, beispielsweise durch eine zusätzliche chemische Vernetzung der Polymermoleküle. Im Grunde wird dadurch allerdings ebenfalls nur die Molekulargewichtsverteilung beeinflußt.

Dieser Weg wird in der internationalen Patentanmeldung WO 86/00814 der Firma Key Pharmaceuticals, Inc. beschrieben. Es wird betont, daß eine solche nachträgliche Vernetzung einem Polyacrylatkleber alleine zwar die Klebrigkeit nimmt, dieser jedoch in Verbindung mit dem in hohen Konzentrationen eingearbeiteten, weichmachenden Wirkstoff - in diesem speziellen Fall Nitroglycerin - gerade eben wieder ausreichend ist bei guter durch die Quervernetzung bewirkter Kohäsion. Diese Lösung des Problems hat allerdings den Nachteil, daß reaktive Substanzen dem Kleber zugemischt werden müssen, deren physiologische Unbedenklichkeit gesichert sein muß.

Zwar sind auch vielen für medizinische Anwendungen geeigneten Polyacrylatklebern physiologisch unbedenkliche Metallchelate (z.b. Titanacetylacetonat, Aluminiumacetylacetonat) zugesetzt, die für eine gewisse Nachvernetzung des Klebers nach Entfernung des Lösemittels sorgen. Diese nachträgliche Vernetzung wird deshalb angewendet, da man dadurch zu Kleberlösungen kommt, die eine geringe Viskosität bei hohem Feststoffanteil besitzen.

Der Grad der so möglichen Nachvernetzung ist aber auf einen Kleber ohne zusätzliche Weichmacher zugeschnitten und kann auch durch eine Erhöhung des Gehalts an solchen Metallchelaten nicht beliebig gesteigert werden. Die Wirkung von Wirkstoffen und/oder Hilfsstoffen kann also nur sehr begrenzt kompensiert werden.

Die EP 0 379 933 (D1) beschreibt ein superficielles therapeutisches System zur lokalen Applikation eines Wirkstoffes, nämlich 5-Fluoruracil. Um bei der Applikation des Wirkstoffes ein bestimmtes Hautareal über längere Zeit zu halten, ohne daß das System seine Haftung zur Haut verliert, wird ein System, bestehend aus einer Rückschicht, einer wirkstoffhaltigen Matrix und einer ablösbaren Schutzschicht vorgeschlagen, wobei die wirkstoffhaltige Matrix folgenden Komponenten enthält:
a) antineoplastischen Wirkstoff
b) ein selbstklebendes Polyacrylat
c) einen Wasserabsorber
   und gegebenenfalls
d) ein nicht klebendes hydrophiles Polyacrylat
e) einen Weichmacher und/oder Penetrationsbeschleuniger.
Die in D 1 aufgeführten Beispiele enthalten als nicht klebendes, hydrophiles Polyacrylat Eudragid RL 100. Das bekannte superficielle therapeutische System nach D1 löst die Aufgabe, aus einem Wundbereich heraus austretende größere Mengen an Körper- bzw. Wundflüssigkeit aufzusaugen, ohne dabei die Kohäsion der Matrix einzubüßen und diese über längere Zeit unter Okklusionsbedinungen zu halten, ohne daß das System seine Haftung zur Haut verliert. Als Lösung wird beansprucht, daß man den polymeren Wirkstoffträger möglichst klar macht und einen zusätzlichen sogenannten Wasserabsorber zusetzt.

Die in D 2, EP 0 288 734 zu lösende Aufgabe besteht darin, ein verbessertes therapeutisches transdermales System zu schaffen, daß komplizierte Änderungen der Wirkstoffabgabe verwirklichen kann. Diese Aufgabe wird durch ein Pflaster gelöst, welche mindestens einen Teil des Wirkstoffreservoirs unter Verbleiben eines oder mehrerer anderer Teile des Wirkstoffreservoirs auf der Haut ablösbar ist, wobei der auf der Haut verbleibende Teil des Wirkstoffreservoirs eine bessere Haftung an der Haut als an der Rückschicht aufweist.

Die DE-A-38 43 239 D3 betrifft ein transdermales therapeutisches System, welches Physostigmin als wirksamen Bestandteil enthält. Damit werden bei Physostigmin auftretende Instabilitäten vermieden und die damit zusammenhängenden Probleme gelöst.

Die EP-A-0 315 219 löst bei einem wirkstoffhaltigen Reservoir die Aufgabe, eine bessere Penetration des Wirkstoffs durch die Hautbarriere zu ermöglichen.

Ausgehend von diesem Stand der Technik liegt der Erfindung die Aufgabe zugrunde, ein transdermales therapeutisches System anzugeben, welches die bei hohen Konzentrationen an weichmachenden Wirk- und/oder Hilfsstoffen auftretenden Konsistenzprobleme beseitigt, ohne daß hierfür ein spezieller Polyacrylatkleber neu synthetisiert, oder ein für sich alleine optimal klebendes Polyacrylat nachträglich chemisch modifiziert werden muß. Es soll demnach der Anwendungsbereich von auf dem Markt befindlichen und möglichst schon in anderen medizinischen Produkten enthaltenen Polyacrylatklebern erweitert werden.

Zur Lösung dieser Aufgabe wird mit der Erfindung bei einer Vorrichtung zur topischen und systemischen Verabreichung von Wirkstoffen an bzw. durch die Haut entsprechend in gattungsbildenden Merkmalen von Anspruch 1 vorgeschlagen, daß die selbstklebende Schicht bzw. Schichten zusätzlich ein klebrigmachendes Harz bevorzugt Phthalsäureester von Hydroabietylalkohol, Glycerinester von hydriertem Colophonium oder niedrigmolekulare Polyacrylate enthält, und daß zumindest einer bzw. der in der selbstklebenden Schicht enthaltenen Hilfsstoff (e) ein Penetrationsbeschleuniger ist.

Die für den Fachmann aus dem Kennzeichnungsteil des Hauptanspruches entnehmbare technische Lehre im Sinne der Schaffung eines transdermalen therapeutischen Präzisionsinstrumentes führt mit der beanspruchten besonderen Zusammensetzung der Vorrichtung zur Lösung des der Erfindung zugrunde liegenden Problems. Dabei hat das filmbildende Polymer aufgrund seines hohen Molekulargewichtes einen positiven Einfluß auf die Kohäsion der Matrix und macht sie außerordentlich aufnahmefähig für weichmachende Inhaltsstoffe. Weichmachende Wirkstoffe und/oder Penetrationsbeschleuniger können bis zu 50 Gew.% bei ausreichender Menge an filmbildendem Polymer eingearbeitet werden. Als gut geeignet erwiesen sich filmbildende Polymere auf der Basis von Polyacryl- bzw. Polymethacrylsäureestern. Besonders gut geeignet sind Copolymerisate von Methacrylsäuremethylester und Methacrylsäurebutylester mit einem mittleren Molekulargewicht von ca. 200.000. Solche Polymethacrylate werden z.B. eingesetzt für sogenannte Sprühpflaster, d.h. sie werden in einem physiologisch unbedenklichen Lösemittel gelöst, aus einer Sprühflasche mittels eines Treibgases direkt auf die Haut und offene Wunden gesprüht und bilden nach dein Verdunstell des Lösemittels einen auf der Haut haftenden Film.

Ein weiterer Vorteil von filmbildenden Polymeren auf dieser Basis ist ihre geringe Wechselwirkung mit Wirk- und/oder Hilfsstoffen, so daß sie keine negativen Auswirkungen auf die Abgabegeschwindigkeit dieser Substanzen haben.

Wenn die Klebkraft der Matrix durch den hohen Gehalt an filmbildendein Polymer zu stark reduziert ist, kann durch den Zusatz von ca. 5 bis 15% stark klebrigmachenden Harzen wirksam Abhilfe geschaffen werden. Als zusätzliche klebrigmachende Zusätze sind alle mit Polyacrylaten verträgliche,, Harze zu verwenden. Als besonders geeignet erwiesen sich Phthalsäureester von Hydroabietylalkohol, Glycerinester von hydriertem Kollophonium und niedermolekulare Polyacrylate.

Zu den weichmachenden Hilfsstoffen zählen beispielsweise die Penetrationsbeschleuniger und Solubilisatoren. Die Auswahl dieser Stoffe wird weitgehend durch die Natur der Wirkstoffe bestimmt, wobei sich als Penetrationsbeschleuniger beispielsweise Fettalkohole, Fettsäureester, Propylenglykol, Ölsäure, Glycerinderivate, Dioctylcyclohexan, N-Methylpyrrolidon oder Caprolactamderivate bewährt haben. Als Solubilisatoren können beispielsweise Propylenglykol, Glycerin, Polyethylenglykol, Ethanol, Ethylacetat oder Acetessigester eingesetzt werden.
Die Gruppe der nicht weichmachenden Hilfsstoffe wird in erster Linie durch Pigmente, wie z.B. Titanoxid und Zinkoxid, Wirkstoffcarrier, wie z.B. Lactose und Kieselgel, Antioxidantien, Trennmittel, wie z.B. Talkum, Puffersubstanzen, wie z.B. anorganische Salze, Glycin und Leucin, und Wasserabsorber gebildet.

In der Regel wird ein einschichtiger Aufbau des Reservoirs die gestellten Anforderungen erfüllen, doch können spezielle Aufgabenstellungen, wie beispielsweise Einstellungen eines Konzentrationsgradienten von Wirk- und/oder Hilfsstoff, eine. mehrschichtige Ausgestaltung des Reservoirs notwendig machen.
Eine bevorzugte Ausgestaltung der erfindungsgemäßen Vorrichtung bei flüssiger oder halbfester Konsistenz des Reservoirs stellt das weiter oben beschriebene sogenannte Beutelsystem dar.

Für alle anderen Bestandteile der Vorrichtung, die in ihrer einfachsten Ausführung nur noch aus einer Rückschicht und einer wieder ablösbaren Schutzschicht besteht, können prinzipiell alle Materialien benutzt werden, die für diesen Zweck bei gewöhnlichen Pflastern, topischen und transdermalen Systemen für den gleichen Verwendungszweck eingesetzt werden.
In dem Falle, wo die Freisetzung der Wirk- und/oder Hilfsstoffe durch eine Membran gesteuert werden muß, ist diese integrierender Bestandteil der Reservoirschicht.

Beispielhaft seien als Materialien für die Rückschicht genannt: flexible oder nicht flexible Folien aus Polyethylen, Polypropylen, Polyethylenterephtalat, Polyvinylchlorid, Polyurethan, Ethylen-Vinylacetat-Copolymere oder Polyamid. Oftmals erreicht man die gewünschten Eigenschaften erst durch den Einsatz von Laminaten aus den oben aufgelisteten Materialien.
Als weitere Materialien können auch Metallfolien wie z.B. Aluminiumfolie oder Papier, allein oder mit einem polymeren Material beschichtet, angewandt werden. Es können auch textile Flächengebilde verwendet werden, wenn die Bestandteile des Reservoirs dieses aufgrund ihrer physikalischen Daten nicht über die Gasphase verlassen können.

Für die wieder entfernbare Schutzfolie können im Prinzip bis auf die textilen Flächengebilde die gleichen Materialien verwendt werden, jedoch müssen sie zusätzlich abhäsiv ausgerüstet sein. Diese abhäsive Ausrüstung kann durch eine spezielle Silikonisierung erreicht werden.

Die mit der erfindungsgemäßen Vorrichtung verabreichbaren Wirkstoffe sind beispielsweise Koronarmittel wie Molsidomin, Nitroglycerin, Isosorbitmononitrat und Isosorbitdinitrat, α1-Rezeptorenblocker wie Prazosin und Terazosin, β-Blocker wie Betaxolol, Pindolol, Timolol, Carteolol und Carazolol, Calciumantagonisten wie Nifedipin und Verapamil, Antiphlogistika und nichtsteroidale Antirheumatika wie Etofenamat, Indometacin, Piroxicam, Acemetacin, Diclofenac, Ibuprofen, Flurbiprofen, Ketoprofen, Carprofen sowie Salicylsäurederivate wie z.B. Ethylenglykolmonosalicylat, Salicylsäuremethylester, Salicylsäureamid oder Phenylsalicylat, Analgetika wie Morphin, Dihydrocodein, Hydromorphon, Oxycodon oder Levomethadon und Hormone wie Estradiol, Levonorgestrel, Norethisteronacetat, Testosteron und 19-Nortestosteron. Weiterhin können erfindungsgemäß auch therapeutisch sinnvolle Kombinationen von Wirkstoffen eingesetzt werden.

So ist beispielsweise zur Behandlung rheumatischer Erkrankungen eine lokale Durchblutungsverbesserung oder Erzeugung eines Wärmegefühls an der zu behandelnden Körperstelle wünschenswert. Dazu kann man den erfindungsgemäßen Pflastern durchblutungsfördernde Stoffe, die ein lokales Wärmegefühl hervorrufen (Rubefacienten, wie Pelargonsäurevanillylamid, Capsaicin, Nicotinsäurederivate, wie Nicotinsäurebenzylester, Nicotinsäuremethylester, Pyridyl-3-carbinol und seine Salze) zusetzen.

Die Erfindung wird durch die nachfolgenden Beispiele erläutert.

### BEISPIELE:

### 1. Pflaster mit Nitroglycerin als Wirkstoff

- 193 g: Polyacrylatkleberlösung
(Durotak 2280-2287, Fa. National Starch, 45% in Ethylacetat)
- 31,9 g: filmbildendes Polyacrylat, Copolymerisat von Methacrylsäuremethylester und Methacrylsäurebutylester
z.B. Plastoid B, Fa. Röhm-Pharma
- 450 g: Nitroglycerinlösung (5% in Chloroform)
- 21 g: Aluminiumacetylacetonatlösung (4% in Ethylacetat)
werden zusammengegeben und homogenisiert. Die flüssige Masse wird 350 µm dick auf 2 verschieden stark silikonisierte Folien gestrichen und 15 Minuten bei 50°C getrocknet. Danach werden die trockenen Filme einmal aufeinanderkaschiert, die stärker silikonisierte Folie entfernt, und das Laminat, z.B. mit einer Polyesterfolie abgedeckt. Die Matrix des so hergestellten transdermalen Systems besitzt ein Flächengewicht von 160 g/m² und einen Gehalt an flüssigem Nitroglycerin von 15 Gew.-%. Unter in-vitro Bedingungen werden pro cm² und 24 h 1,3 mg Nitroglycerin freigesetzt.
Diese Freisetzungsrate ist ausreichend hoch für therapeutische Zwecke.

### 2. Pflaster mit Etofenamat als Wirkstoff

Etofenamat ist bei Raumtemperatur eine ölige Flüssigkeit
- 125 g: Polyacrylatkleberlösung
(Durotak 2280-2287, Fa. National Starch, 45% in Ethylacetat)
- 125 g: filmbildenden Polyacrylat, Copolymerisat von Methacrylsäuremethylester und Methacrylsäurebutylester (50 % w/w in Ethylacetat)
Plastoid B, Fa. Röhm-Pharma
- 90 g: Etofenamat
- 3 g: Aluminiumacetylacetonatlösung (4% in Ethylacetat)
werden zusammengegeben und homogenisiert. Die flüssige Masse wird 200 µm dick auf silikonisierte Folien gestrichen und 20 Minuten bei 50°C getrocknet. Danach wird der trockene Film mit einer Polyesterfolie oder einem textilen Trägergewebe abgedeckt. Die Matrix des so hergestellten transdermalen Systems hat ein Flächengewicht von 90 g/m² und besitzt einen Gehalt an flüssigem Etofenamat von 44 Gew.-%.

Unter in-vitro Bedingungen werden pro cm² und 24 h 0,2 mg Etofenamat freigesetzt. Dies ist eine für therapeutische Zwecke ausreichend schnelle Wirkstoffabgabe.

### 3. Pflaster mit Ethylenglycolmonosalicylat

- 128 g: Copolymerisat von Methacrylsäuremethylester und Methacrylsäurebutylester (50% w/w in Ethylacetat)
z.B. Plastoid B, Fa. Röhm Pharma
- 282 g: Polyacrylatkleber (50% w/w in Ethylacetat)
z.B. Durotak 126-1050, Fa. National Starch
- 59 g: Glycerinester von hydriertem Kollophonium (50% in Ethylacetat)
z.B. Staybelite Ester 5E, Fa. Hercules
- 18 g: Dioctylcyclohexan, z.B. Cetiol S, Fa. Henkel
- 45 g: Ethylenglykolmonosalicylat
- 7,5 g: Pelargonsäurevanillylamid
- 20,2 g: Aluminiumacetylacetonat (4,2% w/w in Ethylacetat)
werden zusammengegeben und homogenisiert. Danach wird auf zwei verschieden stark silikonisierte Folien ein Film gestrichen, der nach 25-minütigem Trocknen bei 50° ein Flächengewicht von 150 g/m² ergibt. Die beiden klebenden Filme werden aufeinanderlaminiert, die stärker silikonisierte Folie entfernt und gegen einen textilen Träger ausgetauscht. Durch Stanzen bzw. Schneiden erhält man die fertigen Pflaster.

### 4. Pflaster mit Ethylenglycolmonosalicylat und Nicotinsäurebenzylester als Wärmekomponente

- 118 g: Filmbildner auf der Basis von Poly(ethylacrylat,, methylmethacrylat, trimethylammonioethylmethacrylatchlorid)
z.B. Eudragit RL, Fa. Röhm Pharma
- 290 g: Polyacrylatkleber (50% w/w in Ethylacetat)
z.B. Durotak 280-2287, Fa. National Starch
- 30 g: Ethylenglykolmonosalicylat
- 3 g: Nicotinsäurebenzylester
- 10 g: Titanacetylacetonat (10% w/w in 2-Propanol)
werden zusammengegeben und homogenisiert. Danach wird auf zwei verschieden stark silikonisierte Folien ein Film gestrichen, der nach 25 minütigem Trocknen bei 50° ein Flächengewicht von 150 g/m² ergibt. Die beiden klebenden Filme werden aufeinander laminiert, die stärker silikonisierte Folie entfernt und gegen einen textilen Träger ausgetauscht. Durch Stanzen bzw. Schneiden erhält man die fertigen Pflaster.

### 5. Pflasterformulierungen mit einem hohen Gehalt an flüssigen Penetrationsbeschleunigern bzw. weichmachenden Zusätzen

Folgende allgemeine Formulierung kann verwendet werden, wenn Pflasterformulierungen gefordert werden, bei denen hohe Konzentrationen an weichmachenden Wirkstoffen und/oder weichmachenden Penetrationsbeschleunigern benötigt werden.
- 100 g: Polyacrylatkleberlösung
(Durotak 2280-2287, Fa. National Starch, 50% in Ethylacetat)
- X g: Lösung eines filmbildenden Polyacrylats
(Plastoid B, Fa. Röhm-Pharma, 50% in Ethylacetat)
- 10 g: Aluminiumacetylacetonatlösung (4% in Ethylacetat)
- Y g: weichmachender Penetrationsbeschleuniger, bzw. Hilfsstoff
werden zusammengegeben und homogenisiert. Die flüssige Masse wird auf silikonisierte Folien 250 µ dick ausgestrichen, 20 Minuten bei 50°C getrocknet und danach mit einer geeigneten Folie abgedeckt.

5.a - 5.e stellen Beispiele dar, die nach der unter 5. angegebenen Vorschrift hergestellt wurden. Die Wirkstoffe sind nicht angegeben, können aber in entsprechenden Mengen zu allen Formulierungen zugegeben werden.

| | Penetrationsbeschleuniger Weichmacher | Gehalt in der Matrix nach Entfernung der Lösemittel |
|---|---|---|
| 5a. | n-Dodecanol X = 29,7; Y = 16 | 20% |
| 5b. | Ölsäure X = 32; Y = 16 | 20% |
| 5c. | Propylenglycol X = 32; Y = 16 | 20% |
| 5d. | Propylenglycol X = 16; Y = 32 | 33% |
| 5e. | N-Methylpyrrolidon X = 16; Y = 32 | 33% |

## Patentansprüche (Patentansprüche für folgende(n) Vertragsstaat(en): AT, BE, CH, DE, DK, FR, GB, IT, LI, LU, NL, SE)

1. Vorrichtung zur topischen und systemischen Verabreichung von Wirkstoffen an bzw. durch die Haut, bestehend aus einer Rückschicht, mindestens einer selbstklebenden Schicht und einer wieder entfernbaren Schutzschicht, wobei die selbstklebende Schicht folgende Bestandteile enthält:
a) 100 Gewichtsteile eines Polyacrylatklebers,
b) 0 bis 250 Gewichtsteile nicht weichmachenden Wirk- und/oder Hilfsstoff,
c) 10 bis 250 Gewichtsteile weichmachenden Wirk- und/oder Hilfsstoff, und wobei dem Polyacrylatkleber 5 bis 150
Gewichtsteile eines mit Polyacrylaten verträglichen filmbildenden Polymeren auf der Basis von Polyacryl- bzw. Polymethacrylsäureestern und bevorzugt von Copolymerisaten von Methacryl-Säuremethylester und Methacrylsäurebutylester zugefügt sind, das für sich alleine nichtklebend ist, aber über sehr gut filmbildende Eigenschaften verfügt, und mit einem mittleren Molekulargewicht von ca. 200.000 einen positiven Einfluß auf die Kohäsion der Matrix hat, diese außerordentlich aufnahmefähig für weichmachende Inhaltsstoffe macht und geringe Wechselwirkung mit Wirk- und/oder Hilfsstoffen hat, dadurch gekennzeichnet, daß die selbstklebende Schicht bzw. Schichten zusätzlich ein klebrigmachendes Harz, bevorzugt Phthalsäureester von Hydroabietylalkohol, Glycerinester von hydriertem Kolophonium oder niedrigmolekulare Polyacrylate enthält, und daß zumindest einer bzw. der in der selbstklebenden Schicht enthaltenen Hilfsstoff (e) ein Penetrationsbeschleuniger ist.

2. Vorrichtung gemäß Anspruch 1 dadurch gekennzeichnet, daß der Wirkstoff Nitroglycerin ist.

3. Vorrichtung gemäß Anspruch 1, dadurch gekennzeichnet, daß der Wirkstoff ein Calciumantagonist, bevorzugt Verapamil ist.

4. Vorrichtung gemäß Anspruch 1, dadurch gekennzeichnet, daß der Wirkstoff ein Antiphlogistikum und/oder Antirheumatikum ist.

5. Vorrichtung gemäß Anspruch 1, dadurch gekennzeichnet, daß der Wirkstoff ein Salicylsäurederivat ist.

6. Vorrichtung gemäß Anspruch 5, dadurch gekennzeichnet, daß das Salicylsäurederivat Ethylenglycolmonosalicylat ist.

7. Vorrichtung gemäß Anspruch 1, dadurch gekennzeichnet, daß der Wirkstoff Etofenamat ist.

8. Vorrichtung gemäß Anspruch 1, dadurch gekennzeichnet, daß der Wirkstoff Indomethacin ist.

9. Vorrichtung gemäß Anspruch 1, dadurch gekennzeichnet, daß der Wirkstoff Ibuprofen ist.

10. Vorrichtung gemäß Anspruch 1, dadurch gekennzeichnet, daß der Wirkstoff Piroxicam ist.

11. Vorrichtung gemäß einem der Ansprüche 1 bis 10, dadurch gekennzeichnet, daß die selbstklebende Schicht einen lokal die Durchblutung fördernden und/oder ein Wärmegefühl hervorrufenden Hilfs- oder Wirkstoff enthält.

12. Vorrichtung gemäß Anspruch 11, dadurch gekennzeichnet, daß dieser Hilfs- bzw. Wirkstoff Pellargonsäurevanillylamid ist.

13. Vorrichtung gemäß Anspruch 11, dadurch gekennzeichnet, daß dieser Hilfs- bzw. Wirkstoff Nicotinsäurebenzylester ist.

14. Vorrichtung gemäß Anspruch 1 bis 13, dadurch gekennzeichnet, daß die Rückschicht aus einem textilen Flächengebilde besteht.

15. Vorrichtung gemäß Anspruch 14, dadurch gekennzeichnet, daß das Flächengebilde wasserabweisend ausgerüstet ist.

16. Vorrichtung gemäß Anspruch 14, dadurch gekennzeichnet, daß das Flächengebilde wasserdampfdurchlässig ist.

17. Vorrichtung gemäß Anspruch 1, dadurch gekennzeichnet, daß der Wirkstoff ein Analgetikum ist.

18. Vorrichtung gemäß Anspruch 1, dadurch gekennzeichnet, daß der Wirkstoff ein Hormon ist.

19. Vorrichtung gemäß Anspruch 1, dadurch gekennzeichnet, daß der Wirkstoff ein Koronarmittel ist.

20. Vorrichtung gemäß Anspruch 1, dadurch gekennzeichnet, daß der Wirkstoff ein Betablocker ist.

21. Vorrichtung gemäß Anspruch 1 bis 20, dadurch gekennzeichnet, daß das System ein Matrixsystem darstellt.

22. Vorrichtung gemäß Anspruch 21, dadurch gekennzeichnet, daß die Matrixschicht mehrschichtig aufgebaut ist.

23. Vorrichtung gemäß Anspruch 22, dadurch gekennzeichnet, daß sie eine die Wirkstoff- und/oder Hilfsstoffabgabe steuernde Membran enhält.

24. Vorrichtung gemäß Anspruch 1 bis 20, dadurch gekennzeichnet, daß das System ein Beutelsystem ist und den Wirkstoff im wesentlichen in einer flüssigen oder halbfesten Zubereitung enthält.

## Patentansprüche (Patentansprüche für folgende(n) Vertragsstaat(en): ES, GR)

1. Verfahren zur Herstellung einer Vorrichtung zur topischen und systemischen Verabreichung von Wirkstoffen an bzw. durch die Haut, bestehend aus einer Rückschicht, mindestens einer selbstklebenden Schicht und einer wieder entfernbaren Schutzschicht, wobei die selbstklebende Schicht folgende Bestandteile enthält:
a) 100 Gewichtsteile eines Polyacrylatklebers,
b) 0 bis 250 Gewichtsteile nicht weichmachenden Wirk- und/oder Hilfsstoff,
c) 10 bis 250 Gewichtsteile weichmachenden Wirk- und/oder Hilfsstoff, und wobei dem Polyacrylatkleber 5 bis 150
Gewichtsteile eines mit Polyacrylaten verträglichen filmbildenden Polieren auf der Basis von Polyacryl- bzw. Polymethacrylsäureestern und bevorzugt von Copolymerisaten von Methacryl-Säuremethylester und Methacrylsäurebutylester zugefügt sind, das für sich alleine nichtklebend ist, aber über sehr gut filmbildende Eigenschaften verfügt, und mit einem mittleren Molekulargewicht von ca. 200.000 einen positiven Einfluß auf die Kohäsion der Matrix hat, diese außerordentlich aufnahmefähig für weichmachende Inhaltsstoffe macht und geringe Wechselwirkung mit Wirk- und/oder Hilfsstoffen hat, dadurch gekennzeichnet, daß zur Erzengung der selbstklebenden Schicht bzw. Schichten zusätzlich ein klebrigmachendes Harz, bevorzugt Phthalsäureester von Hydroabietylalkohol, Glycerinester von hydriertem Kolophonium oder niedrigmolekulare Polyacrylate zugesetzt wird, und daß zumindest als einer bzw. als der in der selbstklebenden Schicht enthaltenen Hilfsstoff(e) ein Penetrationsbeschleuniger zugesetzt wird.

2. Verfahren gemäß Anspruch 1 dadurch gekennzeichnet, daß als Wirkstoff Nitroglycerin zugesetzt wird.

3. Verfahren gemäß Anspruch 1, dadurch gekennzeichnet, daß als Wirkstoff ein Calciumantagonist, bevorzugt Verapamil, zugesetzt wird.

4. Verfahren gemäß Anspruch 1, dadurch gekennzeichnet, daß als Wirkstoff ein Antiphlogistikum und/oder Antirheumatikum zugesetzt wird.

5. Verfahren gemäß Anspruch 1, dadurch gekennzeichnet, daß als Wirkstoff ein Salicylsäurederivat zugesetzt wird.

6. Verfahren gemäß Anspruch 5, dadurch gekennzeichnet, daß als das Salicylsäurederivat Ethylenglycolmonosalicylat zugesetzt wird.

7. Verfahren gemäß Anspruch 1, dadurch gekennzeichnet, daß als Wirkstoff Etofenamat zugesetzt wird.

8. Verfahren gemäß Anspruch 1, dadurch gekennzeichnet, daß als Wirkstoff Indomethacin zugesetzt wird.

9. Verfahren gemäß Anspruch 1, dadurch gekennzeichnet, daß als Wirkstoff Ibuprofen zugesetzt wird.

10. Verfahren gemäß Anspruch 1, dadurch gekennzeichnet, daß als Wirkstoff Piroxicam zugesetzt wird.

11. Verfahren gemäß einem der Ansprüche 1 bis 10, dadurch gekennzeichnet, daß der selbstklebenden Schicht ein lokal die Durchblutung fördernder und/oder ein Wärmegefühl hervorrufender Hilfs- oder Wirkstoff zugesetzt wird.

12. Verfahren gemäß Anspruch 11, dadurch gekennzeichnet, daß als Hilfs- bzw. Wirkstoff Pellargonsäurevanillylamid zugesetzt wird.

13. Verfahren gemäß Anspruch 11, dadurch gekennzeichnet, daß als Hilfs- bzw. Wirkstoff Nicotinsäurebenzylester zugesetzt wird.

14. Verfahren gemäß Anspruch 1 bis 13, dadurch gekennzeichnet, daß als Rückschicht ein textiles Flächengebilde eingesetzt wird.

15. Verfahren gemäß Anspruch 14, dadurch gekennzeichnet, daß das Flächengebilde wasserabweisend ausgerüstet wird.

16. Verfahren gemäß Anspruch 14, dadurch gekennzeichnet, daß ein wasserdampfdurchlässiges. Flächengebilde eingesetzt wird.

17. Verfahren gemäß Anspruch 1, dadurch gekennzeichnet, daß als Wirkstoff ein Analgetikum eingesetzt wird.

18. Verfahren gemäß Anspruch 1, dadurch gekennzeichnet, daß als Wirkstoff ein Hormon eingesetzt wird.

19. Verfahren gemäß Anspruch 1, dadurch gekennzeichnet, daß als Wirkstoff ein Koronarmittel eingesetzt wird.

20. Verfahren gemäß Anspruch 1, dadurch gekennzeichnet, daß als Wirkstoff ein Betablocker eingesetzt wird.

21. Verfahren gemäß Anspruch 1 bis 20, dadurch gekennzeichnet, daß das System als Matrixsystem ausgebildet wird.

22. Verfahren gemäß Anspruch 21, dadurch gekennzeichnet, daß die Matrixschicht mehrschichtig aufgebaut wird.

23. Verfahren gemäß Anspruch 22, dadurch gekennzeichnet, daß in der Vorrichtung eine die Wirkstoff- und/oder Hilfsstoffabgabe steuernde Membran ausgebildet wird.

24. Verfahren gemäß Anspruch 1 bis 20, dadurch gekennzeichnet, daß das System als Beutelsystem ausgebildet wird und den Wirkstoff im wesentlichen in einer flüssigen oder halbfesten Zubereitung enthält.

## Claims (Claims for the following Contracting State(s): AT, BE, CH, DE, DK, FR, GB, IT, LI, LU, NL, SE)

1. Device for the topical and systemic administration of active substances to or through the skin, consisting of a backing layer, at least one self-adhesive layer, and a removable protective layer, whereby said self-adhesive layer contains the following components:
(a) 100 parts by weight of a polyacrylate adhesive,
(b) 0 to 250 parts by weight of a non-softening active substance and/or adjuvant,
(c) 10 to 250 parts by weight of a softening active substance and/or adjuvant, and whereby
5 to 150 parts by weight of a film-forming, polyacrylate-compatible polymer on the basis of polyacrylic acid esters or polymethacrylic acid esters and preferably of copolymerisates of methacrylic acid methyl ester and methacrylic acid butyl ester are added to the polyacrylate adhesive, said polymer on its own being non-adhesive but having very good film-forming properties and, having a mean molecular weight of approximately 200,000, positively affecting the cohesion of the matrix, providing the latter with an extroardinary absorption capacity for softening ingredients, and showing little interaction with active substances and/or adjuvants, **characterised in that** the self-adhesive layer(s) additionally comprise(s) a tackifying resin, preferably phthalic esters of hydroabietyl alcohol, glycerol esters of hydrogenated collophony, or low-molecular polyacrylates, and that at least one of the adjuvants contained in the adhesive layer is a penetration enhancer.

2. The device according to claim 1 wherein the active substance is nitroglycerin.

3. The device according to claim 1 wherein the active substance is a calcium antagonist, preferably verapamil.

4. The device according to claim 1 wherein the active substance is an antiphlogistic and/or an antirheumatic.

5. The device according to claim 1 wherein the active substance is a derivative of salicylic acid.

6. The device according to claim 5 wherein the salicylic acid derivative is ethylene glycol monosalicylate.

7. The device according to claim 1 wherein the active substance is etofenamate.

8. The device according to claim 1 wherein the active substance is indometacin.

9. The device according to claim 1 wherein the active substance is ibuprofen.

10. The device according to claim 1 wherein the active substance is piroxicam.

11. The device according to any one of claims 1 to 10 wherein the self-adhesive layer comprises an adjuvant or active substance which locally stimulates the blood flow and/or creates a feeling of warmth.

12. The device according to claim 11 wherein said adjuvant or active substance is pelargonic acid vanillylamide.

13. The device according to claim 11 wherein said adjuvant or active substance is benzyl nicotinate.

14. The device according to claims 1 to 13 wherein the backing layer consists of a textile fabric.

15. The device according to claim 14 wherein the textile fabric is rendered water-repellent.

16. The device according to claim 14 wherein the textile fabric is permeable to water vapor.

17. The device according to claim 1 wherein the active substance is an analgesic.

18. The device according to claim 1 wherein the active substance is a hormone.

19. The device according to claim 1 wherein the active substance is a coronary stimulant.

20. The device according to claim 1 wherein the active substance is a beta-blocker.

21. The device according to claims 1 to 20 wherein the system is a matrix system.

22. The device according to claim 21 wherein the matrix layer has a multi-layer structure.

23. The device according to claim 22 comprising a membrane controlling the release of active substances and/or adjuvants.

24. The device according to claims 1 to 20 wherein the system is a bag-type system comprising the active substance in a substantially liquid or semisolid preparation.

## Claims (Claims for the following Contracting State(s): ES, GR)

1. Process for producing a device for the topical and systemic administration of active substances to or through the skin, consisting of a backing layer, at least one self-adhesive layer, and a removable protective layer, whereby said self-adhesive layer contains the following components:
(a) 100 parts by weight of a polyacrylate adhesive,
(b) 0 to 250 parts by weight of a non-softening active substance and/or adjuvant,
(c) 10 to 250 parts by weight of a softening active substance and/or adjuvant, and whereby
5 to 150 parts by weight of a film-forming, polyacrylate-compatible polymer on the basis of polyacrylic acid esters or polymethacrylic acid esters and preferably of copolymerisates of methacrylic acid methyl ester and methacrylic acid butyl ester are added to the polyacrylate adhesive, said polymer on its own being non-adhesive but having very good film-forming properties and, having a mean molecular weight of approximately 200,000, positively affecting the cohesion of the matrix, providing the latter with an extroardinary absorption capacity for softening ingredients, and showing little interaction with active substances and/or adjuvants, **characterised in that** for producing the self-adhesive layer(s) a tackifying resin, preferably phthalic esters of hydroabietyl alcohol, glycerol esters of hydrogenated collophony, or low-molecular polyacrylates is additionally added, and that as at least one of the adjuvants contained in the adhesive layer a penetration enhancer is added.

2. The process according to claim 1 wherein nitroglycerin is added as active substance.

3. The process according to claim 1 wherein a calcium antagonist, preferably verapamil, is added as active substance.

4. The process according to claim 1 wherein an antiphlogistic and/or an antirheumatic is added as active substance.

5. The process according to claim 1 wherein a derivative of salicylic acid is added as active substance.

6. The process according to claim 5 wherein ethylene glycol monosalicylate is added as the salicylic acid derivative.

7. The process according to claim 1 wherein etofenamate is added as active substance.

8. The process according to claim 1 wherein indomethacin is added as active substance.

9. The process according to claim 1 wherein ibuprofen is added as active substance.

10. The process according to claim 1 wherein piroxicam is added as active substance.

11. The process according to any one of claims 1 to 10 wherein an adjuvant or active substance which locally stimulates the blood flow and/or creates a feeling of warmth is added to the self-adhesive layer.

12. The process according to claim 11 wherein pelargonic acid vanillylamide is added as the adjuvant or active substance.

13. The process according to claim 11 wherein benzyl nicotinate is added as adjuvant or active substance.

14. The process according to claims 1 to 13 wherein a textile fabric is used as backing layer.

15. The process according to claim 14 wherein the textile fabric is rendered water-repellent.

16. The process according to claim 14 wherein a water vapour-permeable textile fabric is used.

17. The process according to claim 1 wherein an analgesic is used as active substance.

18. The process according to claim 1 wherein a hormone is used as active substance.

19. The process according to claim 1 wherein a coronary stimulant is used as active substance.

20. The process according to claim 1 wherein a beta-blocker is used as active substance.

21. The process according to claims 1 to 20 wherein the system is formed as a matrix system.

22. The process according to claim 21 wherein the matrix layer is provided with a multi-layer structure.

23. The process according to claim 22 wherein a membrane controlling the release of active substances and/or adjuvants is formed in the device.

24. The process according to claims 1 to 20 wherein the system is formed as a bag-type system comprising the active substance in a substantially livid or semisolid preparation.

## Revendications (Revendications pour l'(les) Etat(s) contractant(s) suivant(s): AT, BE, CH, DE, DK, FR, GB, IT, LI, LU, NL, SE)

1. Dispositif pour l'administration topique et systémique de principes actifs à la peau, respectivement par la peau, constitué d'une couche dorsale, d'au moins une couche auto-adhésive et d'une couche de protection détachable, dans lequel la couche auto-adhésive contient les constituants suivants :
a) 100 parts en poids d'une colle de polyacrylate,
b) 0 à 250 parts en poids de principe actif et/ou d'adjuvant non-ramollissant,
c) 10 à 250 parts en poids de principe actif et/ou d'adjuvant ramollissant,
et dans lequel on ajoute à la colle de polyacrylate 5 à 150 parts en poids d'un polymère compatible avec les polyacrylates et formant un film, à base d'esters d'acide polyacrylique, respectivement polyméthacrylique, et de préférence à base de copolymères d'ester méthylique de l'acide méthacrylique et d'ester butylique de l'acide méthacrylique, polymère qui à lui tout seul n'est pas adhésif, mais qui présente de très bonnes caractéristiques de formation d'un film, et qui, avec une masse moléculaire moyenne d'environ 200.000, a un effet positif sur la cohésion de la matrice, qui rend celle-ci extrêmement absorbeuse pour des constituants ramollissants et qui présente une faible interaction avec les principes actifs et/ou les adjuvants, caractérisé en ce que la, respectivement les couche(s) auto-adhésive(s) contien(nen)t en outre une résine qui rend adhésif, de préférence l'ester de l'acide phtalique de l'alcool hydroabiétylique, l'ester de glycérine de colophane hydrogéné ou des polyacrylates inférieurs, et en ce qu'au moins un des adjuvants, respectivement l'adjuvant contenu(s) dans la couche auto-adhésive est un agent d'accélération de la pénétration.

2. Dispositif selon la revendication 1, caractérisé en ce que le principe actif est de la nitroglycérine.

3. Dispositif selon la revendication 1 caractérisé en ce que le principe actif est un antagoniste du calcium, de préférence le vérapamil.

4. Dispositif selon la revendication 1, caractérisé en ce que le principe actif est un agent antiphlogistique et/ou un agent antirhumatique.

5. Dispositif selon la revendication 1, caractérisé en ce que le principe actif est un dérivé de l'acide salicylique.

6. Dispositif selon la revendication 5, caractérisé en ce que le dérivé d'acide salicylique est le monosalicylate d'éthylèneglycol.

7. Dispositif selon la revendication 1, caractérisé en ce que le principe actif est l'étofénamate.

8. Dispositif selon la revendication 1, caractérisé en ce que le principe actif est l'indométacine.

9. Dispositif selon la revendication 1, caractérisé en ce que le principe actif est l'ibuprofène.

10. Dispositif selon la revendication 1, caractérisé en ce que le principe actif est le piroxicam.

11. Dispositif selon une des revendications 1 à 10, caractérisé en ce que la couche auto-adhésive est un adjuvant ou un principe actif favorisant localement la circulation sanguine et/ou provoquant une sensation de chaleur.

12. Dispositif selon la revendication 11 caractérisé en ce que cet adjuvant, respectivement ce principe actif, est l'amide vanillique de l'acide pélargonique.

13. Dispositif selon la revendication 11, caractérisé en ce que cet adjuvant, respectivement ce principe actif, est l'ester benzylique de l'acide nicotinique.

14. Dispositif selon la revendication 1 à 13, caractérisé en ce que la couche dorsale est constituée d'une structure plane texte.

15. Dispositif selon la revendication 14, caractérisé en ce que la structure plane est réalisée sous forme hydrofuge.

16. Dispositif selon la revendication 14, caractérisé en ce que la structure plane est perméable à la vapeur d'eau.

17. Dispositif selon la revendication 1, caractérisé en ce que le principe actif est un agent analgésique.

18. Dispositif selon la revendication 1, caractérisé en ce que le principe actif est une hormone.

19. Dispositif selon la revendication 1, caractérisé en ce que le principe actif est un agent coronaire.

20. Dispositif selon la revendication 1, caractérisé en ce que le principe actif est un bêta-bloquant.

21. Dispositif selon la revendication 1 à 20, caractérisé en ce que le système est un système matriciel.

22. Dispositif selon la revendication 21, caractérisé en ce que la couche de matrice présente une structure multicouche.

23. Dispositif selon la revendication 22, caractérisé en ce qu'il contient une membrane contrôlant la libération de principe actif et/ou d'adjuvant.

24. Dispositif selon la revendication 1 à 20, caractérisé en ce que le système est un système à poche et contient le principe actif essentiellement en une composition liquide ou semi-solide.

## Revendications (Revendications pour l'(les) Etat(s) contractant(s) suivant(s): ES, GR)

1. Procédé de production d'un dispositif pour l'administration topique et systémique de principes actifs à la peau, respectivement par la peau, constitué d'une couche dorsale, d'au moins une couche auto-adhésive et d'une couche de protection détachable, dans lequel la couche auto-adhésive contient les constituants suivants :
a) 100 parts en poids d'une colle de polyacrylate,
b) 0 à 250 parts en poids de principe actif et/ou d'adjuvant non-ramollissant,
c) 10 à 250 parts en poids de principe actif et/ou d'adjuvant ramollissant,
et dans lequel on ajoute à la colle de polyacrylate 5 à 150 parts en poids d'un polymère compatible avec les polyacrylates et formant un film, à base d'esters d'acide polyacrylique, respectivement polyméthacrylique, et de préférence à base de copolymères d'ester méthylique de l'acide méthacrylique et d'ester butylique de l'acide méthacrylique, polymère qui à lui tout seul n'est pas adhésif, mais qui présente de très bonnes caractéristiques de formation d'un film, et qui, avec une masse moléculaire moyenne d'environ 200.000, a un effet positif sur la cohésion de la matrice, qui rend celle-ci extrêmement absorbeuse pour des constituants ramollissants et qui présente une faible interaction avec les principes actifs et/ou les adjuvants, caractérisé en ce que, pour la création de la, respectivement des couche(s) auto-adhésive(s), on ajoute en outre une résine qui rend adhésif, de préférence l'ester de l'acide phtalique de l'alcool hydroabiétylique, l'ester de glycérine de colophane hydrogéné ou des polyacrylates inférieurs, et en ce qu'on ajoute à titre d'au moins un des adjuvants, respectivement à titre de l'adjuvant contenu(s) dans la couche auto-adhésive un agent d'accélération de la pénétration.

2. Procédé selon la revendication 1 caractérisé en ce qu'on ajoute, à titre de principe actif, de la nitroglycérine.

3. Procédé selon la revendication 1, caractérisé en ce qu'on ajoute, à titre de principe actif, un antagoniste du calcium, de préférence le vérapamil.

4. Procédé selon la revendication 1, caractérisé en ce qu'on ajoute, à titre de principe actif, un agent antiphlogistique et/ou un agent antirhumatique.

5. Procédé selon la revendication 1, caractérisé en ce qu'on ajoute, à titre de principe actif, un dérivé de l'acide salicylique.

6. Procédé selon la revendication 5, caractérisé en ce qu'on ajoute à titre de dérivé d'acide salicylique le monosalicylate d'éthylèneglycol.

7. Procédé selon la revendication 1, caractérisé en ce qu'on ajoute, à titre de principe actif, l'étofénamate.

8. Procédé selon la revendication 1, caractérisé en ce qu'on ajoute, à titre de principe actif, l'indométacine.

9. Procédé selon la revendication 1, caractérisé en ce qu'on ajoute, à titre de principe actif, l'ibuprofène.

10. Procédé selon la revendication 1, caractérisé en ce qu'on ajoute, à titre de principe actif, le piroxicam.

11. Procédé selon une des revendications 1 à 10, caractérisé en ce qu'on ajoute à la couche auto-adhésive un adjuvant ou un principe actif favorisant localement la circulation sanguine et/ou provoquant une sensation de chaleur.

12. Procédé selon la revendication 11, caractérisé en ce qu'on ajoute à titre d'adjuvant, respectivement à titre de principe actif, l'amide vanillique de l'acide pélargonique.

13. Procédé selon la revendication 11, caractérisé en ce qu'on ajoute à titre d'adjuvant, respectivement à titre de principe actif, l'ester benzylique de l'acide nicotinique.

14. Procédé selon la revendication 1 à 13, caractérisé en ce qu'on utilise à titre de couche dorsale une structure plane textile.

15. Procédé selon la revendication 14, caractérisé en ce que la structure plane est réalisée sous forme hydrofuge.

16. Procédé selon la revendication 14, caractérisé en ce qu'on utilise une structure plane perméable à la vapeur d'eau.

17. Procédé selon la revendication 1, caractérisé en ce qu'on utilise, à titre de principe actif, un agent analgésique.

18. Procédé selon la revendication 1, caractérisé en ce qu'on utilise, à titre de principe actif, une hormone.

19. Procédé selon la revendication 1, caractérisé en ce qu'on utilise, à titre de principe actif, un agent coronaire.

20. Procédé selon la revendication 1, caractérisé en ce qu'on utilise, à titre de principe actif, un bêta-bloquant.

21. Procédé selon la revendication 1 à 20, caractérisé en ce que le système est réalisé sous forme de système matriciel.

22. Procédé selon la revendication 21, caractérisé en ce que la couche de matrice présente une structure multicouche.

23. Procédé selon la revendication 22, caractérisé en ce qu'on réalise dans ce dispositif une membrane contrôlant la libération de principe actif et/ou d'adjuvant.

24. Procédé selon la revendication 1 à 20, caractérisé en ce que le système est réalisé sous forme d'un système à poche et contient le principe actif essentiellement en une composition liquide ou semi-solide.
